(19) Europäisches Patentamt
European Patent Office
Office européen des brevets

(11) Publication number: **0 145 054 B1**

# (12) EUROPEAN PATENT SPECIFICATION

(45) Date of publication of patent specification: **23.03.88**

(51) Int. Cl.$^4$: **C 07 C 126/02**

(21) Application number: **84201600.8**

(22) Date of filing: **06.11.84**

## (54) Process for preparing urea.

(30) Priority: **13.11.83 NL 8303888**

(43) Date of publication of application: **19.06.85 Bulletin 85/25**

(45) Publication of the grant of the patent: **23.03.88 Bulletin 88/12**

(84) Designated Contracting States: **AT BE DE FR GB IT NL SE**

(56) References cited:
**EP-A-0 093 466**
**GB-A-1 091 925**
**GB-A-1 505 445**
**GB-A-1 539 009**
**GB-A-2 083 471**
**GB-A-2 083 472**
**US-A-3 147 304**
**US-A-3 867 442**

(73) Proprietor: **STAMICARBON B.V.**
**Mijnweg 1**
**NL-6167 AC Geleen (NL)**

(72) Inventor: **Jonckers, Kees**
**Illikhoven 13**
**NL-6121 RH Born (NL)**

(74) Representative: **Hoogstraten, Willem Cornelis Roeland et al**
**OCTROOIBUREAU DSM Postbus 9**
**NL-6160 MA Geleen (NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 145 054 B1

**Description**

The invention relates to a process for preparing urea from ammonia and carbon dioxide.

If ammonia and carbon dioxide are fed into a synthesis zone under a suitable pressure (for instance 125—350 atm) and at a suitable temperature (for instance 170—250°C), first ammonium carbamate is formed, in conformity with the reaction:

$$2\ NH_3+CO_2 \rightarrow H_2N—CO—ONH_4$$

Subsequently, from the resulting ammonium carbamate urea is formed by dehydration according to the reaction:

$$H_2N—CO—ONH_4 \rightleftarrows H_2N—CO—NH_2+H_2O$$

The degree to which the conversion into urea proceeds depends, among other things, on the temperature and the excess of ammonia that is used. The resulting reaction product in this process is a solution substantially consisting of urea, water, ammonium carbamate and uncombined ammonia. The ammonium carbamate and the ammonia must be removed from the solution and are returned in most cases to the synthesis zone. The synthesis zone may consist of separate zones for the formation of carbamate and urea. These zones, however, may also be combined in one apparatus.

A mode frequently applied for preparing urea is described in European Chemical News, Urea Supplement, of 17th January 1969, pages 17—20. In that process the urea synthesis solution formed in the synthesis zone at high pressure and temperature is stripped at synthesis pressure bij contacting the solution countercurrently with gaseous carbon dioxide while supplying heat, so that the larger part of the carbamate present in the solution is decomposed into ammonia and carbon dioxide, and these decomposition products are driven off in gaseous form from the solution and carried off along with a small amount of water vapour and the carbon dioxide used for the stripping. Besides using carbon dioxide, as described in that publication, the stripping can be done also with gaseous ammonia, an inert gas or with a mixture of at least two of the said gases. The heat required for the stripping is obtained by condensation of high pressure steam of 15—25 bar on the shell side of the tubes of the vertical heat exchanger in which the stripping is effected. The gas mixture obtained in the stripping process is condensed for the larger part in a first condensation zone and absorbed in an aqueous solution originating from the further treatment of the urea-containing solution, upon which the aqueous carbamate solution formed in that process as well as the non-condensed gas mixture are carried to the synthesis zone for the formation of urea. Here the heat required for the conversion of carbamate into urea is obtained by further condensation of the gas mixture.

The stripped urea synthesis solution is subsequently expanded to a low pressure of for instance 2—6 bar and heated by means of steam in order to remove from the solution ammonia and carbon dioxide still partly present in the form of carbamate in the stripped urea solution. The resulting gas mixture, which also contains water vapour, is condensed and absorbed in an aqueous solution in a second condensation zone operating at low pressure and the dilute carbamate solution formed in that process is pumped back to the high pressure section of the urea synthesis and eventually passed into the synthesis zone. The remaining urea-containing solution is subjected to further expansion and upgraded to form a urea solution or melt that may be further processed to form solid urea. To this end the aqueous urea solution is evaporated usually in two evaporation steps and the resulting urea melt is processed to form granules, or the urea solution is crystallized. The gases obtained in the evaporation or crystallization, which gases contain in addition to water vapour, ammonia, carbon dioxide and entrained fine urea drops, are condensed while so-called process condensate is being formed. A part of the process condensate is used as absorbent for the gas mixture in the second condensation zone. The remaining part can be treated with high pressure steam for the purpose of decomposing urea contained therein into ammonia and carbon dioxide and recovering these decomposition products along with the ammonia and carbon dioxide already present as such.

It has already been proposed to introduce into such a process an extra decomposition step in which further amounts of carbamate still present in the stripped urea synthesis solution are decomposed at a pressure of 12—25 kg/cm$^2$ (see the British patent application 2,083,472). The heat released in the condensation of the gas mixture obtained in the extra decompostion step is carried off by means of a urea crystal suspension passed through the condensation zone via cooling tubes, the heat required for the evaporation of water being transferred to the crystal suspension. The circulation of a crystal suspension with pumps through cooling tubes is a disadvantage, because the presence of solid particles gives rise to disturbances in the process operation, while, moreover, erosion of the cooling tubes may occur. Moreover, in the process described, the heat absorbed by the crystal suspension is utilized only partly in the crystallization process, because a part of the crystals present is carried off as product.

It has already also been proposed to use the heat released in the condensation of a gas mixture containing ammonia and carbon dioxide for the concentration of a urea solution (see the British patent specification 1,091,925). This known process does not comprise a stripping treatment, but constitutes a process in which the urea synthesis solution is expanded and heated in two pressure steps and the gases released in this expansion are separated from the solution. The

solution obtained in the first pressure step after separating off the gas mixture released in the expansion is heated and the gas mixture formed in this operation is subjected to heat exchange with the urea solution to be evaporated, this solution being concentrated thereby. The gas mixture cools down in this treatment and is condensed partly.

It has now been found that in the evaporation of urea solutions more economic use can be made of the heat released in the condensation of ammonia- and carbon dioxide-containing gas mixtures obtained in the urea preparation process at a sufficiently high level of pressure if the dew point of the mixture to be condensed is raised so that in the heat exchange the gas mixture condenses virtually completely. Thus it is not only the sensible heat, but also the heat of dissolution and the heat of condensation that can be utilized almost completely.

The object of the invention is to provide a process for preparing urea, in which process economic use is made, during the evaporation of the eventually resulting urea solution, of heat that can be developed in the further processing of the stripped urea synthesis solution. This is achieved according to the invention by decomposing the carbamate still present in the stripped urea synthesis solution in at least two steps of a decreasing level of pressure, completely condensing the gases separated off in the first of these steps and transferring the heat released in this process to the urea solution to be evaporated. In order to achieve virtually complete condensation, the dew point of the gas mixture to be condensed is raised by adding water or an aqueous solution and the gas-liquid mixture formed in this process is subjected to heat exchange with the urea solution to be evaporated.

The invention consequently relates to a process for preparing urea in which,

—in a synthesis zone, at a pressure of 125—350 bar and at the temperature belonging thereto, a urea synthesis solution containing carbamate and free ammonia is formed from carbon dioxide and an excess of ammonia,

—in a first decomposition step, at synthesis pressure or lower, a part of the carbamate is decomposed by supply of heat and the gas phase obtained in this process is condensed in a first condensation zone,

—in a second decomposition step, at a pressure of 4—40 bar, a further part of the carbamate still present is decomposed by supply of heat and the gas phase formed is separated off,

—in a further step, the remaining carbamate is removed in the form of a gas mixture containing ammonia and carbon dioxide, and this gas mixture is processed to form a solution of ammonia and carbon dioxide in water,

—and the remaining urea-containing solution is processed to form a concentrated urea solution.

This process is characterized in that the urea solution to be concentrated is introduced into the tubes of a shell and tube heat exchanger, the gas mixture from the second decomposition step is led into the shell side of heat exchanger at the end opposite the place where the urea solution to be concentrated is fed, the solution of ammonia and carbon dioxide in water is fed to the said shell side at a place located between the inlet for the gas mixture and the inlet for the urea solution to be concentrated, and the condensate is discharged from the shell side at the end where the urea solution is led into the tubes. In this way it is achieved that the dew point of the gas to be condensed is increased without the first portion of the evolving heat of condensation of the supplied gas mixture being used to heat the supplied dilute carbamate solution, which has a lower temperature; instead, all of this heat can be used to heat the urea solution to be evaporated. In doing so, it is advantageous to lead the solution of ammonia and carbon dioxide in water into the shell side of the heat exchanger at a place where the temperature in the shell side substantially corresponds to the temperature at which, in a Q—t-diagram, the condensation line of the gas mixture in the absence of the dilute carbamate solution and the condensation line of the gas mixture in the presence of the dilute carbamate solution intersect, Q being the amount of heat transferred from the shell side to the tube side and t being the temperature in the shell side.

With the combination of measures proposed here, it is possible to make very efficient use of the sensible heat as well as the heat of condensation and the heat of absorption of the gas mixture from the second decomposition step.

At relatively low pressures in the second decomposition step, within the limits indicated, this results mainly in an increase in the amount of exchangeable heat. At relatively high pressures in the second decomposition step, within the limits indicated, mainly an increase of the temperature difference between the shell side and the tube side of the heat exchanger is achieved, so that, for a given amount of heat to be transferred, a smaller heat-transfer area will do.

At higher pressures in the second decomposition step, the amount of gas mixture to be condensed, and hence also the amount of heat released in condensation is smaller, and additional steam-heating of the urea solution may be necessary. Therefore, in general the pressure in the second decomposition step will be kept below 25 bar, more in particular between 15 and 22 bar.

In that case, a third decomposition step operated under a pressure of 1—10 bar is desired. However, it is also possible to operate the second decomposition step under a pressure of between 4 and 10 bar. In this case, the urea solution obtained in this step can be sent directly to the concentration step, where decomposition of the remaining carbamate and concentration then take place simultaneously.

If the decomposition of carbamate not converted to urea takes place in three steps, it is advantageous to lead the gas mixture to be

condensed into the heat exchanger with a dew point of between 110 and 160°C, so that here, too, the temperature difference between the condensing gas and the urea solution to be evaporated in the top of the heat exchanger is above the minimum value at which heat transfer takes place. As a rule, it will suffice that the temperature of the gas mixture is between 125 and 180°C.

The process according to the invention can be applied in processes for preparation of urea in which the carbamate is decomposed by a stripping treatment followed by heating in one or more pressure steps as well as in processes in which carbamate decomposition takes place by heating in a number of pressure steps only.

The invention will be elucidated with reference to the figure and the example without, however, being restricted there to.

In the embodiment according to the figure, 1 indicates a synthesis zone, 2 represents a first decomposition step, for instance a stripping zone, 3 a first condensation zone and 4 a scrubber. These apparatuses form part of the high-pressure part of the urea synthesis in which a pressure of, for instance, 125—350 bar is maintained. With 5, 8 and 10 expansion valves are indicated, with 6 a heating zone, 7 and 9 represent gas-liquid separators, 11 is a storage tank for the urea solution to be evaporated. With 12 and 13 are indicated, respectively, the first and second evaporation steps, 14 represents a second condensation zone and 15 a storage tank for the carbamate solution obtained in the heat exchange in the first evaporation step. 16 represents a pump for pumping carbamate solution and 17 a liquid ejector.

Through 18 and ejector 17 liquid ammonia is passed, together with the carbamate solution supplied through 19 from scrubber 4, through 20 into the first condensation zone 3. Through 21 the gas mixture from stripping zone 2 is fed to it as well. This gas mixture is obtained by passing in stripping zone 2, while supplying of heat, the urea synthesis solution carried off through 22 from synthesis zone 1 countercurrently to carbon dioxide introduced into this zone through 27.

This first condensation zone may, for instance, be designed as a vertical shell and tube heat exchanger. The heat released in this zone in the formation of ammonium carbamate is carried off by means of boiler feed water, which is converted in this process into low-pressure steam of 4—5 bar. The stripping zone, too, can be designed in the form of a vertical shell and tube heat exchanger. The heat required for the stripping is supplied in the form of high-pressure steam of, for instance, 15—30 bar. The carbamate solution formed in the first condensation zone 3 and the non-condensed gas are supplied to the synthesis zone 1 through 23. In this zone enough heat is developed by the further condensation of ammonia and carbon dioxide to carbamate to cover the heat requirements of the endothermic conversion of carbamate into urea. Through 24 the gas mixture not condensed in the synthesis zone, which gas mixture contains the inert gases introduced into the process with the fresh ammonia and carbon dioxide and possibly the passivating air or oxygen, is supplied to scrubber 4, where ammonia and carbon dioxide present in the gas are scrubbed out with carbamate solution supplied through 26. The inert gases are discharged through 25.

The stripped urea synthesis solution is carried off from stripping zone 2 and passed through 28, expansion valve 5, in which the pressure is lowered to 4—40 bar, for instance about 24 bar, and via heating zone 6, in which the reaction mixture is heated up by heat exchanger to 125—180°C, for instance about 165°C, in which treatment a part of the carbamate still present is decomposed, to gas-liquid separator 7. From this separator the gas phase formed, a mixture containing substantially ammonia, carbon dioxide and water vapour, is carried off through 29 and the remaining liquid phase through 30. By means of expansion valve 8 the pressure of the liquid phase is lowered to 1—10 bar, for instance about 7 bar, and the gas-liquid mixture formed in this process is passed to gas-liquid separator 9. The gas phase released in it, a mixture containing substantially ammonia, carbon dioxide and water vapour, is passed through 31 to the second condensation zone 14 and condensed there to form a carbamate solution with the aid of an aqueous solution, for example process condensate, supplied via 41. The heat released in the condensation is carried off by means of cooling water. The liquid phase obtained in gas-liquid separator 9 flows through 32 and expansion valve 10, in which the solution is expanded to atmospheric pressure or lower, for instance about 0.6 bar, to a storage tank 11. From this storage tank the resulting solution, containing for instance about 70% (wt) urea, which solution still contains ammonia and carbon dioxide, is supplied through 34 to the first evaporation step 12. Subsequently, the urea solution already concentrated to, for instance, more than 90% (wt) urea is passed through 35 to the second evaporation step 13. The heating zone of these evaporation steps is for the process of the invention preferably designed as a vertical shell and tube heat exchanger. In principle, also a horizontal shell and tube heat exchanger can be used, but this requires a more complex construction of the apparatus. The water vapour obtained in the two evaporation steps, containing small amounts of liquid urea, ammonia and carbon dioxide, is sent after condensation through respectively 39 and 40 to a non-represented installation for the processing of process condensate, like the gas phase carried off through 38 from storage tank 11.

The heat required for the evaporation in the second evaporation step 13 is supplied by condensation of low-pressure steam. The heat required in the first evaporation step 12 is supplied by condensation of the gas mixture carried off through 29 from gas-liquid separator 7. To this end this gas mixture is passed into the shell side

of the shell and tube heat exchanger of the first evaporation step 12, to which the carbamate solution from the second condensation zone 14 is supplied simultaneously through 33. Thus the dew point of the gas mixture to be condensed is raised. The carbamate solution also functions as condensing agent and solvent for the gas mixture to be condensed.

Preference is given to passing the gas-liquid mixture through the heating zone countercurrently to the urea solution to be evaporated. The carbamate solution formed in the shell side in the condensation process is passed through 36 to a storage stank 15, brought under synthesis pressure by pump 16 and passed through 26 into the top of scrubber 4 for the removal of ammonia and carbon dioxide from the gas mixture containing the inert gases, which has been carried off through 24 from synthesis zone 1.

The evaporated urea solution is carried off through 37.

If the heat released in the condensation of the gas mixture supplied through 29 does not suffice to meet the total heat requirements of the first evaporation step, the deficiency can be supplied by condensation of low-pressure steam. In that case the shell side of the heating zone must be divided into two comparments.

Example 1

Using the process described, urea is prepared according to the embodiment represented in the figure in an installation having a production capacity of 1500 tonnes a day. The quantities are given in kg per hour. The pressure applied in the high-pressure section of the installation is 157.3 bar, in the second decomposition step and in the shell side of the heater of the first evaporation step 23.5 bar.

To the high-pressure section of the installation 25,560 kg $NH_3$, which contains 107 kg $H_2O$ and which has been preheated to 85°C, is supplied and 38,681 kg of a carbamate solution containing 16,521 kg $CO_2$, 13,392 kg $NH_3$ and 8,228 kg $H_2O$. The temperature in the reaction zone is 183°C and the molar N/C ratio 2.95. To the stripping zone 179,594 kg urea synthesis solution is supplied and stripped with 48,133 kg gas mixture containing 46,079 kg $CO_2$ and for the rest consisting of inert gases, mainly air. From the stripping zone 120,382 kg urea, 291 kg biuret, 15,556 kg $CO_2$, 13,193 kg $NH_3$, 27,775 kg $H_2O$ and 27 kg inert components are carried off. The pressure of the stripped urea synthesis solution is lowered to 23.5 bar and by heat exchange the temperature of the expanded mixture is brought to 165°C. Thus, in gas-liquid separator 7,24,183 kg of a gas mixture is obtained containing 14,681 kg $CO_2$, 7,062 kg $NH_3$, 2,413 kg $H_2O$ and 27 kg inert. In addition 96,199 kg of a liquid phase remains containing 63,539 kg urea, 291 kg biuret, 876 kg $CO_2$, 6,131 kg $NH_3$ and 25,362 kg $H_2O$. The pressure of this liquid phase is lowered to 6.9 bar and the resulting mixture is passed into gas-liquid separator 9. Here 4,392 kg of a gas mixture consisting of 552 kg $CO_2$, 2,612 kg $NH_3$ and 1,228 kg $H_2O$ is separated off. The pressure of the remaining solution is further lowered to 0.65 bar, which yields 84,663 kg urea solution with a temperature of 90°C consisting of 63,477 kg urea, 324 kg biuret, 24 kg $CO_2$, 593 kg $NH_3$ and 20,245 kg $H_2O$. The gas mixture obtained in gas-liquid separator 9 is condensed in the second condensation zone by means of a carbamate solution formed in the working up of the process condensate, which carbamate solution contains 2,059 kg $CO_2$ 2,875 kg $NH_3$ and 4,623 kg $H_2O$. In the second condensation zone 15,750 kg solution with a temperature of 45°C is obtained consisting of 2,611 kg $CO_2$, 7,289 kg $NH_3$ and 5,850 kg $H_2O$. This solution is brought under a pressure of 23.5 bar and fed together with the gas mixture from gas-liquid separator, into the top of the shell side of the heating section of the first evaporating step, countercurrently to the urea solution to be evaporated. The gas mixture is then condensed, in which process 38,681 kg carbamate solution with a temperature of 124°C is formed. The urea solution passed through the tubes of the heating section is concentrated in this process and leaves the heater with a temperature of 130°C. The composition of this solution is 67,874 kg urea, 425 kg biuret, 216 kg $NH_3$ and 3,379 kg $H_2O$. From the top of the first evaporation step 17,798 kg vapour mixture escapes consisting of 16,774 kg $H_2O$, 275 kg $CO_2$, 581 kg $NH_3$, 23 kg inert and 146 kg urea. If the heating of the first evaporation step is effected with low-pressure steam, 18,390 kg steam of 4 bar is required to obtain the same result.

Example 2

Urea is prepared according to the embodiment represented in the figure in an installation having a production capacity of 1000 tonnes a day. The quantities are given in kg per hour.

In the second decomposition step, in which a pressure of 18 bar is maintained, a gas mixture is obtained containing 9,440 kg $CO_2$, 5,680 kg $NH_3$ and 1,810 kg $H_2O$.

The temperature of this gas mixture is 153°C its dew point is 139°C.

This gas mixture is condensed with the aid of a carbamate solution having a temperature of 45°C and containing 1,470 kg $CO_2$, 4,440 kg $NH_3$ and 4,470 kg $H_2O$ in the shell side of a vertical shell and tube heat exchanger which forms the heating section of the first evaporation step. To this end the gas mixture is fed into the top of the shell side, countercurrently to the urea containing solution to be evaporated containing 41,667 kg urea, 132 kg $CO_2$, 1,343 kg $NH_3$ and 14,639 kg $H_2O$. The urea containing solution which is passed through the tubes of the heating section is concentrated by the heat released thereby to a 95 wt.-% urea solution and leaves the heating section with a temperature of 130°C. If the heating of the first evaporation step is effected with low pressure steam, 13,100 kg steam of 4 bar is required to obtain the same result.

**Claims**

1. Process for preparing urea in which,

—in a synthesis zone, at a pressure of 125—350 bar and at the temperature belonging thereto, a urea synthesis solution containing carbamate and free ammonia is formed from carbon dioxide and an excess of ammonia,

—in a first decomposition step, at synthesis pressure or lower, a part of the carbamate is decomposed by supply of heat and the gas phase obtained in this process is condensed in a first condensation zone,

—in a second decomposition step, at a pressure of 4—40 bar, a further part of the carbamate still present is decomposed by supply of heat and the gas phase formed is separated off,

—in a further step, the remaining carbamate is removed in the form of a gas mixture containing ammonia and carbon dioxide, and this gas mixture is processed to form a solution of ammonia and carbon dioxide in water,

—and the remaining urea-containing solution is processed to form a concentrated urea solution,

characterized in that the urea solution to be concentrated is introduced into the tubes of a shell and tube heat exchanger, the gas mixture from the second decomposition step is led into the shell side of the heat exchanger at the end opposite the place where the urea solution to be concentrated is fed, the solution of ammonia and carbon dioxide in water is fed to the said shell side at a place located between the inlet for the gas mixture and the inlet for the urea solution to be concentrated, and the condensate is discharged from the shell side at the end where the urea solution is led into the tubes.

2. Process according to Claim 1, characterized in that the solution of ammonia and carbon dioxide in water is led into the shell side of the heat exchanger at a place where the temperature in the shell side substantially corresponds to the temperature at which, in a Q—t-diagram, the condensation line of the gas mixture in the absence of the dilute carbamate solution and the condensation line of the gas mixture in the presence of the dilute carbamate solution intersect, Q being the amount of heat transferred from the shell side to the tube side and t being the temperature in the shell side.

3. Process according to claim 1 or 2, characterized in that the gas mixture to be condensed has a dew point of between 110 and 160°C when it is led into the shell side of the heat exchanger.

**Patentansprüche**

1. Verfahren zur Herstellung von Harnstoff, worin

—in einer Synthesezone bei einem Druck von 125—350 bar und der dazugehörigen Temperatur eines Harnstoffsyntheselösung enthaltend Carbamat und freies Ammoniak aus Kohlendioxid und einem Überschuß an Ammoniak gebildet wird,

—in einer ersten Zersetzungsstufe bei Synthesedruck oder niedriger ein Teil des Carbamats durch Zufuhr von Wärme zersetzt wird und die bei diesem Verfahren erhaltene Gasphase in einer ersten Kondensationszone kondensiert wird,

—in einer zweiten Zersetzungsstufe bei einem Druck von 4—40 bar ein weiterer Teil des noch vorhandenen Carbamats durch Zufuhr von Wärme zersetzt wird und die gebildete Gasphase abgetrennt wird,

—in einer weiteren Stufe das verbleibende Carbamat in Form eines Gasgemisches, das Ammoniak und Kohlendioxid enthält, entfernt wird und dieses Gasgemisch unter Bildung einer Lösung von Ammoniak und Kohlendioxid in Wasser verarbeitet wird,

—und die harnstoffhaltige Lösung zur Bildung einer konzentrierten Harnstofflösung verarbeitet wird,

dadurch gekennzeichnet, daß die zu konzentrierende Harnstofflösung in die Rohre eines Röhrenbündelwärmeaustauschers eingeführt wird, das Gasgemisch von der zweiten Zersetzungsstufe in die Mantelseite des Wärmeaustauschers an dem Ende gegenüber der Stelle, wo die zu konzentrierende Harnstofflösung eingeführt wird, geführt wird, die Lösung von Ammoniak und Kohlendioxid in Wasser zu der genannten Mantelseite geführt wird an einer Stelle, die zwischen dem Einlaß für das Gasgemisch und dem Einlaß für die zu konzentrierende Harnstofflösung lieft, und das Kondensat von der Mantelseite an dem Ende, wo die Harnstofflösung in die Rohre eingeführt wird, abgezogen wird.

2. Verfahren gemäß Anspruch 1, dadurch gekennzeichnet, daß die Lösung von Ammoniak und Kohlendioxid in Wasser in die Mantelseite des Wärmeaustauschers an einer Stelle eingeführt wird, wo die Temperatur in der Mantelseite im wesentlichen der Temperatur entspricht, bei der in einem Q—t-Diagramm die Kondensationslinie des Gasgemisches in Abwesenheit von verdünnter Carbamatlösung und die Kondensationslinie des Gasgemisches in Anwesenheit der verdünnten Carbamatlösung sich schneiden, wobei Q die Wärmemenge ist, die von der Mantelseite zu der Röhrenseite übertragen wird, und t die Temperatur in der Mantelseite ist.

3. Verfahren gemäß Anspruch 1 oder 2, dadurch gekennzeichnet, daß das zu kondensierende Gasgemisch einen Taupunkt zwischen 110 und 160°C hat, wenn es in die Mantelseite des Wärmeaustauschers geführt wird.

**Revendications**

1. Procédé pour la préparation d'urée dans lequel:

—dans une zone de synthèse, sous une pression de 125—350 bars et à la température correspondante, on forme une solution de synthèse de l'urée contenant du carbamate et de l'ammoniac libre, à partir de gaz carbonique et d'un excès d'ammoniac;

—dans une première opération de décomposition, à la pression de synthèse ou au-dessous,

une portion du carbamate est décomposée par apport de chaleur et la phase gazeuse produite dans ce processus est condensée dans une première zone de condensation;

—dans une deuxième opération de décomposition, sous une pression de 4—40 bars, une autre portion du carbamate encore présent est décomposée par apport de chaleur et la phase gazeuse formée est séparée;

—dans une opération suivante, le carbamate restant est éliminé sous forme d'un mélange gazeux contenant de l'ammoniac et du gaz carbonique, ce mélange gazeux est traité pour former une solution d'ammoniac et de gaz carbonique dans l'eau;

—et la solution subsistante contenant l'urée est traitée pour former une solution d'urée concentrée;

caractérisé en ce que la solution d'urée qui doit être concentrée est introduite dans les tubes d'un échangeur de chaleur à tubes et enveloppe, le mélange gazeux de la seconde opération de décomposition est introduit du côté enveloppe de l'échangeur de chaleur à l'extrémité opposée de l'endroit où la solution d'urée à concentrer est introduite, la solution d'ammoniac et de gaz carbonique dans l'eau est introduite dudit côté enveloppe à l'endroite placé entre l'entrée du mélange gazeux et l'entrée de la solution d'urée qui doit être concentrée, et le condensat est déchargé du côté enveloppe à l'extrémité où la solution d'urée est introduite dans les tubes.

2. Procédé selon la revendication 1, caractérisé en ce que la solution d'ammoniac et de gaz carbonique dans l'eau est introduit du côté enveloppe de l'échangeur de chaleur à un endroit où la température du côté enveloppe correspond pratiquement à la température à laquelle, dans un diagramme Q—t, la ligne de condensation du mélange gazeaux en l'absence de solution de carbamate dilué et la ligne de condensation du mélange gazeux en présence d'une solution de carbamate dilué se coupent, Q étant la quantité de chaleur transférée du côté enveloppe vers le côté tubes et t étant la température dans le côté enveloppe.

3. Procédé selon l'une des revendications 1 et 2, caractérisé en ce que le mélange gazeux à condenser a un point de rosée compris entre 110 et 160°C lorsqu'il est introduit dans le côté enveloppe de l'échangeur de chaleur.

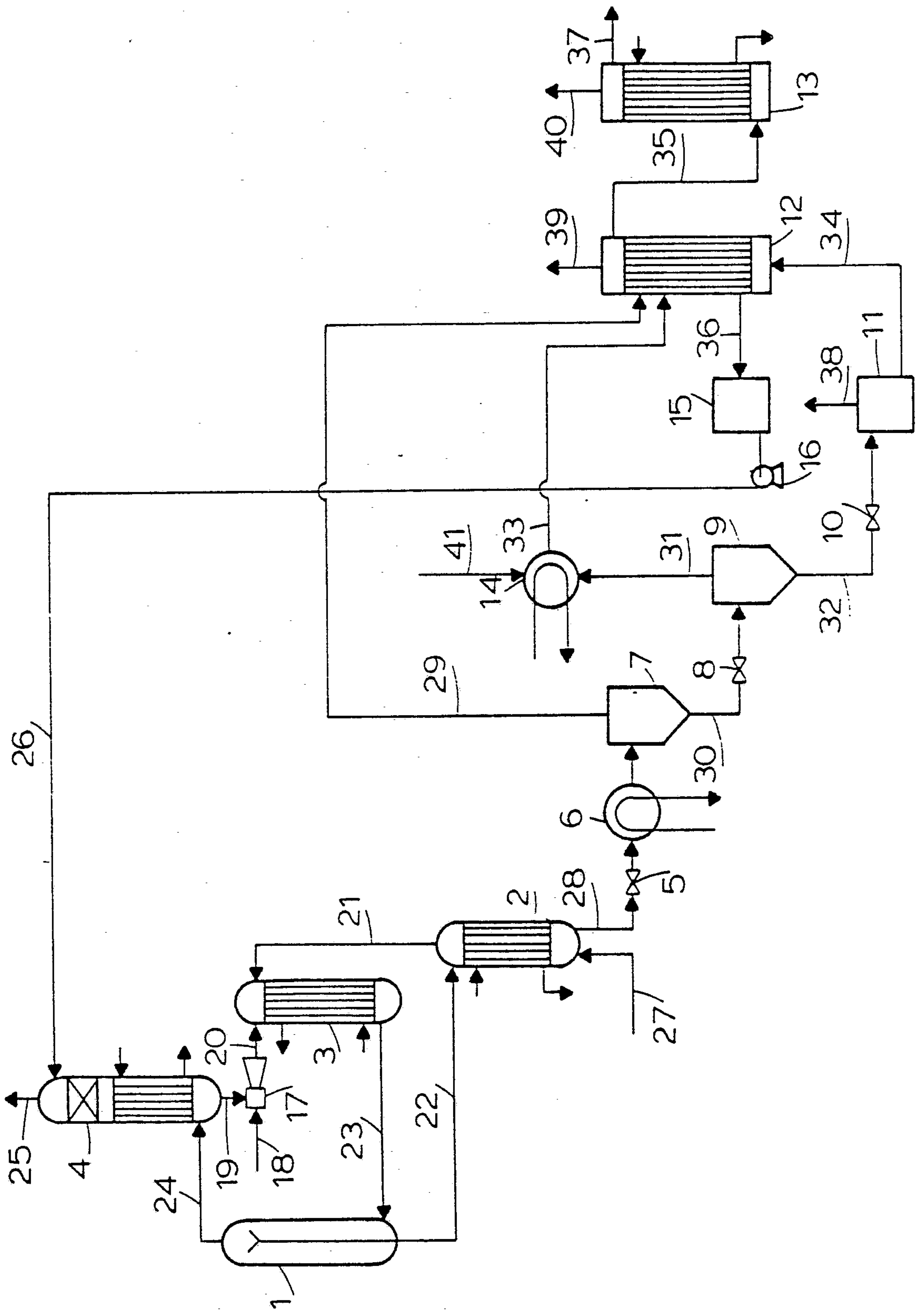
1
2
3
4
5
6
7
8
9
10
11
12
13
14
15
16
17
18
19
20
21
22
23
24
25
26
27
28
29
30
31
32
33
34
35
36
37
38
39
40
41